# EUROPEAN PATENT APPLICATION

(11) **EP 1 338 280 A1**
(43) Date of publication of application: **27.08.2003**
(21) Application number: 01983613.9
(22) Date of filing: 06.11.2001
(51) Int. Cl.: A61K 31/28, A61K 31/13, A61P 3/10

(54) **COMBINATION OF AMINES AND VANADIUM (IV)/(V) COMPOUNDS FOR THE TREATMENT AND/OR PREVENTION OF DIABETES MELLITUS**

(30) Priority: 07.11.2000 ES 200002745
(71) Applicant: Universidad de Barcelona, 08028 Barcelona (ES)
(72) Inventor: ZORZANO OLARTE, Antonio Ctr de Pa. Univ. de Barc., E-08028 Barcelona (ES); CARPENE, Christian Ctr de Pat. Univ. de Barc., E-08028 Barcelona (ES); ENRIQUE-TARANCON DELMAS, Gemma, E-08028 barcelona (ES); MARTI, Luc Ctr de Pat. Universidad de Barcelona, E-08028 Barcelona (ES); TESTAR YMBERT, Xavier Ctr de Pat. Univ. de Barc., E-08028 Barcelona (ES); PALACIN PRIETO, Manuel Ctr de Pat. Univ. de Barc., E-08028 Barcelona (ES); ABELLA MARTI, Anna Ctr de Pat. Univ. de Barc., E-08028 Barcelona (ES)
(74) Representative: Graf von Stosch, Andreas, Dr. rer.nat.
(86) International application number: ES0100424
(87) International publication number: WO02038152

(57) **Abstract**

Combinations comprising vanadium (IV)/(V) compounds and pharmaceutically acceptable amines selected from the group of semicarbazide-sensitive amine oxidase (SSAO) substrates are insulin mimickers. Preferred vanadium (IV)/(V) compounds are vanadyl salts, vanadyl complexes and vanadates (e.g. sodium orthovanadate). Preferred amines are tyramine and benzylamine. A here-discovered sinergism between the vanadium compound and the amine makes the effective concentration of vanadate in the combination one order of magnitude lower than the corresponding of vanadate alone. Consequentely the combination has much lower toxicity than the known vanadium compound alone, what is a crucial advantage of the former for its use in the treatment and/or prevention of Diabetes mellitus.

## Description

This invention relates to pharmaceutical compositions comprising a combination of certain amines and pharmaceutically acceptable vanadium (IV)/(V) compounds, for use as insulin mimickers, i.e., to fight against the illness Diabetes mellitus.

### DESCRIPTION OF THE PRIOR ART

Diabetes mellitus is a major global health problem which is recognised by the World Health Organisation to be reaching epidemic proportions. It is now the fourth leading cause of death in most developed countries and a disease that is increasing rapidly in countries undergoing industrialisation. Diabetes mellitus is a disease caused by defective carbohydrate metabolism and characterized by abnormally large amounts of sugar glucose in the blood and urine. Diabetes mellitus can eventually damage the eyes, kidneys, heart, and limbs, and can endanger pregnancy.

Diabetes mellitus is usually classified into two types. Type 1, or insulin-dependent Diabetes mellitus (IDDM), formerly called juvenile-onset diabetes, which occurs in children and young adults, has been implicated as one of the autoimmune diseases. Rapid in onset and progress, it accounts for about 10 to 15 percent of all cases. Type 2, or non-insulin-dependent Diabetes mellitus (NIDDM), formerly called adult-onset diabetes, is usually found in persons over 40 years old and progresses slowly. Type 2, which is by far the most frequent one, is often not accompanied by clinical illness in its initial stages and is detected instead by elevated blood or urine glucose levels.

Diabetes is considered a group of disorders with multiple causes, rather than a single disorder. The human pancreas secretes a hormone called insulin that facilitates the entry of the sugar glucose into tissues of the body and its utilization, thus providing energy for bodily activities. In a person with diabetes, however, the entry of glucose is impaired, a result either of a deficiency in the amount of insulin produced or of altered target cells. Consequently, sugar builds up in the blood and is excreted in the urine. In the Type 1 diabetic, the problem is almost always a severe or total reduction in insulin production. In the Type 2 diabetic, the pancreas often makes a considerable quantity of insulin, but the hormone is unable to promote the utilization of glucose by tissues. In fact, a central feature of Type 2 diabetes is the existence of insulin resistance. The provision of new insulin-mimickers for the treatment and/or prevention of diabetes is a therapeutical problem, and this problem is specially important in relation to the most frequent kind of the illness, i.e. diabetes Type 2.

Semicarbazide-sensitive amine oxidase (SSAO) encompasses a group of enzymes showing a broad tissue distribution in mammals, but whose biological function is not known. To date, SSAO substrates have not been proposed for the treatment of Diabetes mellitus, neither alone, nor in combination with other agents. In fact, SSAO has never been associated with an insulin-mimicker activity.

In recent years, several inorganic compounds have been described which mimic the effects of insulin, both in vivo and in isolated cells and tissues. These include vanadium (IV)/(V) compounds (cf. Heyliger et al., "Effect of vanadate on elevated blood glucose and depressed cardiac performance of diabetic rats", Science 1985, vol. 227, pp. 1474-7); selenate (cf. McNeill et al., "Insulinlike effects of sodium selenate in streptozotrocin-induced diabetic rats", Diabetes 1991, vol. 40, pp. 1675-8), lithium salts (cf. Rodriguez-Gil et al., "Lithium restores glycogen synthesis from glucose in hepatocytes from diabetic rats", Arch. Biochem. Biophys. 1993, vol. 301, pp. 411-5), and tungsten (VI) compounds (cf. US 5,595,763).

Among the vanadium derivatives which have been studied as insulin-mimickers the following ones are included: vanadates and peroxovanadium complexes (vanadium in its +5 oxidation state combined with oxygen, specially orthovanadate VO₄³⁻, cf. US 4,882,171), and vanadyl VO²⁺ salts and complexes (vanadium in its +4 oxidation state; cf. US 5,300,496). Inventors of the present invention have shown that some combinations of benzylamine or tyramine (two SSAO substrates) and low concentrations of vanadate stimulates glucose transport in rat adipocytes (cf. G. Enrique-Tarancon et al., Biochem. J. 2000, vol. 350, pp. 171-180). But there is nothing in the art pointing to a similar activity in muscle and beta-pancreatic cells from diabetic subjects, i.e., pointing to the use of those combinations against Diabetes.

Vanadium compounds are currently undergoing clinical trials in Europe and America. But administration of vanadium compounds has the drawback of its toxicity at effective doses. Administered concentrations must be close to the toxic level, if the insulin-mimetic effects in animals are to be achieved. Vanadium-treatment is always accompanied by marked negative side effects that are independent of the chemical form of vanadium used (cf. Domingo et al., "Oral vanadium administration to streptozocin-diabetic rats has marked negative side-effects wich are independent of the form of vanadium used", Toxicology 1991, voL 66, pp. 279-87.). Remarkable signs of vanadium compounds toxicity are observed at all the doses that are able to lower blood glucose, incluiding a significant mortality rate. Therefore, a substantial reduction in doses of vanadium compounds, while keeping their insulin-mimicker activity; is an important problem in the therapy and/or prevention of Diabetes mellitus.

### SUMMARY OF THE INVENTION

This invention solves the above-mentioned problem by providing a new pharmaceutical combination comprising a pharmaceutically acceptable vanadium (IV)/(V) compound and a pharmaceutically acceptable amine selected from the group of semicarbazide-sensitive amine oxidase (SSAO) substrates, in admixture with pharmaceutically acceptable excipients or carriers. Said combination is useful for the treatment and/or prevention of Diabetes mellitus in mammals, particularly in humans. Depending of circumstances to be determined in every case by usual pharmacological and clinical tests, the active ingredients of the combination can be administered simultaneously, separatedly or sequentially. Although parenteral administration is possible, the oral one is preferred.

In the context of this invention, the term "a pharmaceutically acceptable vanadium (IV)/(V) compound" is intended to include any chemical entity formed by one or several vanadium atoms in its +4 or its +5 oxidation states, attached to a chemical structure that is pharmaceutically acceptable by itself. The cations V⁴⁺ and V⁵⁺ have never been observed isolated, and they come always accompanied with a chemical moiety partially formed by a coordination sphere. The coordination sphere can be formed by inorganic ligands (oxide, hydroxide, peroxide, etc) as, for example, in the case of the orthovanadate anion VO₄³⁻ (vanadium +5 and a coordination sphere formed by four oxide ions), and in the case of vanadyl cation VO²⁺ (vanadium +4 and a coordination sphere formed by one oxide ions). The coordination sphere can also be formed by organic ligands which are molecules or ions attached to the vanadium atoms through O, S or N atoms belonging to different pharmaceutically acceptable organic compounds (e.g. pharmaceutically acceptable alcohols, tiols, carboxilyc acids, amines, aminoacids, N-containing heterocycles, etc). Mixed inorganic/organic coordination spheres are also possible as, for example, in peroxovanadium complex ions [VO(O₂)₂L-L']ⁿ⁻, L-L' being a bi-dentate organic ligand such as 1,10-phenanthroline. When the structure formed by the vanadium atom and its coordination sphere is not neutral, the term "chemical moiety" also includes any pharmaceutically aceptable ionic species which makes neutral the whole compound. For example, vanadate anions are always accompanied by cations (e.g. sodium, potassium, magnesium, calcium) to form neutral salts. The term "pharmaceutically acceptable vanadium (IV)/(V) compound" also include any pharmaceutically acceptable solvate (e.g. hydrate) of said compound".

The term "semicarbazide-sensitive amine oxidase" (SSAO) is intended to mean any enzyme present in tissue of mammals which is able to oxidize a broad variety of amines with the concomitant production of hydrogen peroxide, and which is inhibited by semicarbazide. With a few examples of secondary amines, most of the substrates of semicarbazide-sensitive amine oxidases are primary amines, such as: tyramine, benzylamine, deoxyepinephrine, epinephrine, norepinephrine, dopamine, histamine, β-phenylethylamine, N-acetylputrescine, tryptamine, n-octylamine, n-pentylamine, kynuramine, 3-methoxytyramine, and n-decylamine.

In a preferred embodiment, the pharmaceutical combination comprises a vanadium compound selected from the group consisting of vanadates, peroxovanadium complexes, vanadyl salts and vanadyl complexes. Specially preferred are those containing a vanadate, and more preferred those with sodium orthovanadate Na₃VO₄.

In another preferred embodiment, the pharmaceutical composition contains an amine selected from the group consisting of tyramine, benzylamine, deoxyepinephrine, epinephrine, norepinephrine, dopamine, histamine, β-phenylethylamine, N-acetylputrescine, tryptamine, n-octylamine, n-pentylamine, kynuramine, 3-methoxytyramine, and n-decylamine. Specially preferred are the combination containing tyramine or benzylamine. The amine can also be in the form of a salt with any of the pharmaceutically acceptable organic or inorganic acids known in the art.

Another aspect of the present invention relates to the treatment and/or prevention of Diabetes mellitus in a mammal, specially in a human, comprising the administration of any of the above-mentioned pharmaceutical combinations. It is also part of the present invention the use of a mixture of a pharmaceutically acceptable vanadium (IV)/(V) compound and a pharmaceutically acceptable amine of the SSAO substrates group, or a salt thereof, for the preparation of a medicament for the treatment and/or prevention of Diabetes mellitus.

Examples 1-4 illustrate that the combinations of the present invention are insulin mimickers. Thus, the combination of vanadate and tyramine stimulate glucose transport (Example 1) and glucose transporter translocation (Example 2) in the same way as insulin does. Similarly, the combination of vanadate and benzylamine stimulate glucose transport (Example 3) and glucose transporter translocation (Example 4) in the same way as insulin does. Example 5 illustrates that the above-mentioned activities are not limited to tyramine or to benzylamine; in fact, it is a general property of amines which are SSAO substrates.

For the purpose of the present invention it is crucial that the effective concentrations of vanadate in the insulin-mimicker combinations are one order of magnitude lower (or less) than the concentrations needed to mimic insulin action when vanadate is alone. This means that there is synergism between the vanadium compound and the amine. From a practical point of view, such synergism means that, for a given insulin mimicker effect, the toxicity of the drug based on a combination vanadium+amine is much lower than the toxicity of a drug based on vanadium alone. This represents an important advantage of the pharmaceutical combination of the present invention in respect of the vanadium compositions known in the art, for the treatment and/or prevention of Diabetes mellitus.

The in the specification and the claims used term "contains" and its variations, as well as the term "containing" does not exclude other additives, components, elements or steps. The disclosure in the summary, which is attached to this application, and in the priority application, is incorporated by reference as well. The following examples and figures serve for illustration purposes and are by no means limiting for the present invention.

### BRIEF DESCRIPTION OF THE DRAWINGS

The results are, in part, set out graphically in the following figures:
FIG. 1 is a graphical illustration of the variation of glucose transport values (expressed as percentage of maximal values, E) in isolated rat adipocites, due to a 45 min incubation in the presence of different concentrations (expressed as logarithm of molar concentration, log [M]) of insulin (filled triangles), hydrogen peroxide (filled circles) and three substrates of semicarbazide-sensitive amine oxidase (tyramine, open diamonds; serotonin, open squares; phenylephrine, open circles).
FIG. 2 is a graphical illustration of the variation of glucose transport values (expressed as concentration C of 2-deoxyglucose in nanomoles, taken up in five minutes, per 100 mg of lipid) in isolated rat adipocites due to a 45 min incubation in the following conditions: A, basal; B, insulin 0.1 µM; C, hydrogen peroxyde 1.0 mM; D, tyramine 1.0 mM. Striped bars correspond to incubation in the presence of 0.1 mM sodium orthovanadate; open bars correspond to control incubation. Numbers of observations are shown in parenthesis.
FIG. 3 is a graphical illustration of the variation of glucose transport values (expressed as concentration C of 2-deoxyglucose in nanomoles, taken up in ten minutes, per 100 mg of lipid) in isolated rat adipocites due to a 45 min incubation in the presence of different agents: (a) insulin 0.1 µM; (b) benzylamine 0.1 mM; (c) sodium orthovanadate 0.1 mM; (d) semicarbazide 1.0 mM. Each bar corresponds to a different set of conditions, where a plus sign indicates the presence of the corresponding agent, and a minus sign indicates its absence.
FIG. 4 is a graphical illustration of the variation of glucose transport values (expressed as percentage E of the maximal effect of insulin) in isolated rat adipocites due to a 45 min incubation in different conditions and different amines. (A)/(B) correspond to incubations performed in the absence/presence of sodium vanadate 0.1 mM. Filled/open bars correspond to incubations performed in the presence of 1.0 mM/0.1 mM concentration of amine. Every low case letter corresponds to a different amine, as follows: (a) deoxyepinephrine; (b) epinephrine; (c) dopamine; (d) norepinephrine; (e) tyramine; (f) histamine; (g) β-phenylethylamine; (h) N-acetylputrescine; (i) tryptamine; (j) n-octylamine; (k) n-pentylamine; (l) kynuramine; (m) 3-methoxytyramine; (n) benzylamine and (o) n-decylamine.
FIG. 5 is a graphical illustration of the variation of glycemia (G, expressed in mg/dl) versus the day of treatment (d). Diabetic rats were treated by a daily i.p. injection of vanadate (50 µmol/kg) alone (open circles) or in combination with benzylamine delivered by osmotic minipumps (84 µmol/kg/day) (open squares). Sham-operated diabetic rats that received a daily injection of the vehicle served as control (solid diamonds). Values are mean ± SEM of 4-12 observations.
FIG. 6 and FIG. 7 are graphical illustrations of the variation of glycemia (G, expressed in mg/dl) versus the day of treatment (d) for rats injected i.p. daily with vanadate at 25 µmol/kg body wt during the first week and at 50 µmol/kg body wt during the second week. Benzylamine-treated diabetic rats (open triangles), vanadate-treated diabetic rats (open circles) and benzylamine and vanadate-treated diabetic rats (open squares). Values are mean ± SEM of 3-6 observations per group.

### EXAMPLES

### 0. General methods

Adipocyte isolation. Male Wistar rats (200-260 g) were sacrificed in a fed state, and the internal epididymal adipose tissues was dissected out and digested in Krebs-Ringer buffer as described (cf. C. Carpene et al., Biochem. PharmacoL 1990, voL 40, pp. 437-445). Adipose cells isolated is such a way were used in subsequent studies.

Determination of amine oxidase activity. Amine oxidase activity was measured as described (cf. P.H. Yu, "Monoamine oxidase", in A.A. Boulton et aL eds., "Neuromethods, Neurotransmitter Enzymes", 1986, voL 5, pp. 235-272, Humana Press, Clifton, NJ, USA), using [¹⁴C]tyramine or [¹⁴C]benzylamine as substrates.

Hexose transport After a preincubation period of 45 min at 37 °C in vials containing 400 µl of cell suspension in KRBH with the tested products, [³H]-2-deoxyglucose was added at a final concentration of 0.1 mM (approx. 1,300,000 d.p.m./vial). The assays were further incubated for 5 or 10 min and then stopped with 100 µl of cytochalasin B. Aliquots (200 µl) of the cell suspension were centrifuged in microtubes as described (cf. J.M. Olefsky, Biochem. J. 1978, voL 172, pp. 137-145). After centrifugation, the fat cells (upper part of the tubes) were placed in scintillation vials and the incorporated radioactivity was counted. The extracellular 2-deoxyglucose (2-DG) present in the cell fraction was determined using adipocytes whose transport activity had been previously blocked by cytochalasin B. It did not exceed 1 % of the maximum 2-DG uptake in the presence of insulin.

### Example 1: Potentiation by orthovanadate of tyramine effect on 2-deoxyglucose uptake

Whether amine oxidation could mimic the action of insulin on glucose transport through the generation of hydrogen peroxide was tested. It was checked that glucose transport was not activated after 45 min preincubation of fat cells with increasing concentrations of tyramine, serotonin or phenylephrine whereas, in the same conditions (5-min 2-DG uptake), insulin increased basal uptake twelve-fold (Figure 1). However, exogenously added H₂O₂ dose-dependently stimulated hexose transport (filled circles in Figure 1). When preincubation lasted 45 min, 1 mM H₂O₂ multiplied the basal transport by a 5.0 ± 0.8 fold factor, an effect equivalent to 46 % that of the maximal insulin effect (n = 23).

Figure 2 shows that orthovanadate potentiates the effect of tyramine as well as that of hydrogen peroxide. Orthovanadate alone (0.1 mM) did not affect basal or insulin-stimulated glucose transport, whereas, when combined with 1 mM hydrogen peroxide it provoked a substantial activation equivalent to three quarters of the maximal effect of insulin. On a larger number of observations (n = 40), the stimulation of glucose transport induced by 1 mM tyramine plus 0.1 mM orthovanadate reached 54 ± 5 % that of 100 nM insulin whilst orthovanadate alone reached only 5 ± 1 % of the maximal insulin effect (orthovanadate plus insulin giving the same response as insulin alone: 102 ± 3 %, n = 40). Moreover, there was no synergism between tyramine and low, ineffective, doses of insulin (10 pM) or hydrogen peroxide (0.01 mM) (not shown).

When present at 0.1 mM, semicarbazide inhibited 59 ± 7 % of the glucose transport promoted by 1 mM tyramine plus orthovanadate. Glucose transport stimulated by 1 mM hydrogen peroxide plus 0.1 mM orthovanadate was unaffected by 1 mM semicarbazide.

### Example 2: GLUT4 translocation induced by tyramine plus orthovanadate.

To verify whether the stimulation of glucose transport in rat adipocytes may be explained by a translocation of the insulin-sensitive glucose transporters from an internal site to the plasma membrane, as it is known for insulin, the amount of GLUT4 protein was determined in plasma membrane (PM) and low density microsome (LDM) fractions. Western blot analysis showed that, in conditions where insulin increased the amount of GLUT4 protein in the PM by 252 ± 43 % (n = 3), tyramine plus orthovanadate increased it by 163 ± 9 % (around half the insulin effect) whereas orthovanadate at 0.1 mM alone was inefficient (103 ± 8 %). These increases in GLUT4 content occurred without any significant change in the content of β1-integrin, a constitutive PM protein. Changes in the GLUT4 content of LDM mirrored, to a lesser extent, those reported in the PM (basal LDM content was decreased by 29 % and 12 % with insulin and tyramine plus orthovanadate respectively).

### Example 3: Synergic stimulation of glucose transport by benzylamine and orthovanadate in isolated rat adipocytes

The effect of benzylamine, a preferential substrate of SSAO, on 2-deoxyglucose uptake by isolated rat adipocytes is shown in Figure 3. The results depicted in the Figure show that, under these conditions, there is no stimulatory effect of benzylamine (100 mM). However, the combination of benzylamine (100 mM) and a very low concentration of orthovanadate (100 mM), which alone did not stimulate 2-deoxyglucose uptake, caused a near 6-fold stimulation of glucose transport. Furthermore, the benzylamine-orthovanadate-stimulated 2-deoxyglucose uptake was dependent on the activity of SSAO; thus, incubation in the presence of semicarbazide (1 mM), the specific inhibitor of SSAO activity abolished the stimulation of 2-deoxyglucose uptake by benzylamine and orthovanadate.

### Example 4: Recruitment of GLUT4 glucose carriers to the plasma membrane in adipose cells, by benzylamine and orthovanadate

This study was carried out in order to determine whether the stimulation of glucose transport induced by the combination of benzylamine and orthovanadate was a consequence of alterations in the abundance of GLUT4 glucose carriers in the cell surface from adipose cells. Thus, isolated rat adipocytes were incubated in the absence or in the presence of insulin, benzylamine (100 mM), orthovanadate (100 mM) or the combination of benzylamine and orthovanadate, and 45 minutes later cells were homogenized and subjected to subcellular membrane fractionation. It was observed that insulin treatment caused a 3.3-fold increase in the abundance of GLUT4 in plasma membrane preparations and that, under these conditions, incubation of cells with benzylamine or orthovanadate alone did not alter the abundance of GLUT4 in plasma membranes. However, the incubation of cells with benzylamine and orthovanadate caused a 2.3-fold increase in the presence of GLUT4 in plasma membrane fractions. The effects of insulin or the combination benzylamine and orthovanadate on GLUT4 abundance in plasma membranes were specific and the plasma membrane marker β₁-integrin remained unaltered after incubation with these agents. The recruitment of GLUT4 to the plasma membranes detected by insulin or the combination of benzylamine and orthovanadate was concomitant with a reduction of GLUT4 abundance in light microsomes. This was consistent with the view that benzylamine and orthovanadate translocate GLUT4 to the plasma membrane in isolated rat adipocytes.

### Example 5: Activity of other substrates of semicarbazide-sensitive amine oxidase

Figure 4 shows the stimulatory effects of different substrates of semicarbazide-sensitive amine oxidase on glucose transport by isolated rat adipocites. It is observed that fifteen different primary amines which are substrates of semicarbazide-sensitive amine oxidase, show a sinergic stimulation of glucose tansport in the presence of 0.1 mM orthovanadate. This indicates that the effect is general among that kind of substrates.

### Example 6: Chronic administration of benzylamine and vanadate reduces hyperglycemia in diabetic rats.

Based on the finding of potent insulin-like effects of in vitro incubation of benzylamine and vanadate, the chronic administration of these compounds in diabetic rats was tested. To this end, streptozotocin-induced diabetic rats were implanted subcutaneously with osmotic minipumps releasing benzylamine (84 µmol/kg/day) or were sham-operated. Preliminary studies indicated that benzylamine was stable for two weeks in implanted osmotic minipumps as based on its capacity to further stimulate glucose transport in isolated adipocytes after this period. A group of animals was also subjected to daily i.p. injection of vanadate (50 µmol/kg body wt) for two weeks. While treatment with vanadate caused a moderate reduction of glycemia the combination of benzylamine and vanadate normalized glycemia after one week of treatment (FIG. 5). Vanadate-benzylamine combination values (marked with *) were significantly different from sham-operated and vanadate-only groups from day 6, at P < 0.05. In addition, administration of benzylamine plus vanadate for two weeks did not alter SSAO activity in extracts from adipose cells.

In other studies, rats were daily injected intraperitoneally with 25 µmol/kg vanadate for the first week and with 50 µmol/kg for the second week. These rats, as well as diabetic rats treated with 84 µmol/kg benzylamine alone remained hyperglycemic (FIG. 6). However, the combination of benzylamine and vanadate reduced hyperglycemia in diabetic rats during the second week of treatment (FIG. 7). Vanadate-benzylamine combination values (marked with *) were significantly different compared with vanadate group from day 5, at P < 0.05.

The chronic treatment with benzylamine and vanadate caused a substantial decrease in food and water consumption which returned to normal levels and an increase in the weight of epididymal adipose tissue. All these variations occurred in the absence of changes in body weight. The effects on adiposity or food and water intake were not detected when benzylamine or vanadate was administered alone. Furthermore the normalisation of glycemia caused by chronic treatment with both benzylamine and vanadate takes place in the absence of changes in plasma insulin concentrations.

## Claims

1. An antidiabetic combination for simultaneous, separate or sequential administration, comprising a pharmaceutically acceptable vanadium (IV)/(V) compound and a pharmaceutically acceptable amine selected from the group consisting of semicarbazide-sensitive amine oxidase (SSAO) substrates, or a pharmaceutically acceptable salt of said amine, in admixture with pharmaceutically acceptable excipients or carriers.

2. A combination according to claim 1, wherein the vanadium compound is selected from the group consisting of vanadates, peroxovanadium complexes, vanadyl salts and vanadyl complexes.

3. A combination according to claim 2, wherein the vanadium compound is selected from the group of vanadates.

4. A combination according to claim 3, wherein the vanadium compound is sodium orthovanadate.

5. A combination according to any of the claims 1-4, wherein the amine is selected from the group consisting of tyramine, benzylamine, deoxyepinephrine, epinephrine, norepinephrine, dopamine, histamine, β-phenylethylamine, N-acetylputrescine, tryptamine, n-octylamine, n-pentylamine, kynuramine, 3-methoxytyramine, and n-decylamine.

6. A combination according to claim 5, wherein the amine is tyramine.

7. A combination according to claim 5, wherein the amine is benzylamine.

8. Use of a pharmaceutically acceptable vanadium (IV)/(V) compound and a pharmaceutically acceptable amine selected from the group consisting of semicarbazide-sensitive amine oxidase (SSAO) substrates, or a pharmaceutically acceptable salt of said amine, for the preparation of a medicament for the treatment and/or prevention of Diabetes mellitus, by simultaneous, separate or sequential administration.

9. Use according to claim 8, wherein the vanadium compound is selected from the group consisting of vanadates, peroxovanadium complexes, vanadyl salts and vanadyl complexes.

10. Use according to claim 9, wherein the vanadium compound is selected from the group of vanadates.

11. Use according to claim 10, wherein the vanadium compound is sodium orthovanadate.

12. Use according to any of the claims 8-11, wherein the amine is selected from the group consisting of tyramine, benzylamine, deoxyepinephrine, epinephrine, norepinephrine, dopamine, histamine, β-phenylethylamine, N-acetylputrescine, tryptamine, n-octylamine, n-pentylamine, kynuramine, 3-methoxytyramine, and n-decylamine.

13. Use according to claim 12, wherein the amine is tyramine.

14. Use according to claim 12, wherein the amine is benzylamine.

15. Product containing a pharmaceutically acceptable vanadium (IV)/(V) compound and a pharmaceutically acceptable amine selected from the group of semicarbazide-sensitive amine oxidase (SSAO) substrates, or a pharmaceutically acceptable salt of said amine, as a combined preparation for simultaneous, separate or sequential administration, for the treatment and/or prevention of Diabetes mellitus.

16. Product according to claim 15, wherein the vanadium compound is selected from the group consisting of vanadates, peroxovanadium complexes, vanadyl salts and vanadyl complexes.

17. Product according to claim 16, wherein the vanadium compound is selected from the group of vanadates.

18. Product according to claim 17, wherein the vanadium compound is sodium orthovanadate.

19. Product according to any of the claims 15-18, wherein the amine is selected from the group consisting of tyramine, benzylamine, deoxyepinephrine, epinephrine, norepinephrine, dopamine, histamine, β-phenylethylamine, N-acetylputrescine, tryptamine, n-octylamine, n-pentylamine, kynuramine, 3-methoxytyramine, and n-decylamine.

20. Product according to claim 19, wherein the amine is tyramine.

21. Product according to claim 19, wherein the amine is benzylamine.
